# EUROPEAN PATENT SPECIFICATION

(11) **EP 3 064 172 B1**
(45) Date of publication and mention of the grant of the patent: **05.10.2022**
(21) Application number: 14858484.0
(22) Date of filing: 31.10.2014
(51) Int. Cl.: A61F 2/07, A61F 2/848

(54) **COATED STENT GRAFT**
BESCHICHTETER STENT-GRAFT
ENDOPROTHÈSE COUVERTE

(30) Priority: 31.10.2013 CN 201310534126
(43) Date of publication of application: 07.09.2016
(73) Proprietor: Shanghai MicroPort Endovascular MedTech (Group) Co., Ltd., Shanghai 201318 (CN)
(72) Inventor: LI, Zhonghua, Shanghai 201203 (CN); ZHU, Qing, Shanghai 201203 (CN); WANG, Liwen, Shanghai 201203 (CN); XING, Zhikai, Shanghai 201203 (CN); ZHU, Yongfeng, Shanghai 201203 (CN); YAN, Dongmei, Shanghai 201203 (CN)
(74) Representative: Lavoix
(86) International application number: PCT/CN2014/089968
(87) International publication number: WO 2015/062531

(56) References cited:
- EP-A1- 1 839 624
- EP-A1- 2 301 476
- WO-A1-97/09007
- WO-A1-99/39662
- CN-A- 102 048 596
- US-A1- 2003 135 261
- US-A1- 2006 224 232
- US-A1- 2011 218 609

## Description

### TECHNICAL FIELD

The invention relates to a stent graft, which can be used for treating the abdominal aortic aneurysm.

### BACKGROUND

At present, there are many kinds of stent grafts for treating the abdominal aortic aneurysm and the iliac aneurysm on the market, and the various products have different advantages and disadvantages, and have different treatment points. The stent graft for treating the abdominal aortic aneurysm should mainly have the following characteristics: the stent graft should be adapted to a bent tumor neck and a smaller tumor neck length, should have comparatively good adhesion and roundness properties, should have comparatively good bending properties, should have a moderate support strength, should be capable of preventing a long-term displacement and the like.

At present, on the domestic and international markets, there are mainly the following performance indexes to evaluate this kind of stent grafts: ① tightness: evaluating the validity of the stent isolating the tumor cavity from the blood; ② anti-displacement: preventing the displacement of the stent after the placement of the stent; ③ release resistance: a proper release resistance being capable of smoothly releasing the stent and avoiding the displacement of the stent; ④ positioning accuracy: evaluating the degree of offset between the positioning before the release and the position of the stent graft after the release; ⑤ stent flexibility: evaluating the ability of the stent graft being adaptively bent in a bent blood vessel; ⑥ adaptation to a bent tumor neck; and ⑦ a smaller tumor neck length.

Multiple stent grafts are disclosed in the prior art. For example, the patent document EP2301476A1 discloses a suturing and knotting method for a stent graft, in which this knotting method is used for fixing the stent section and the graft tube to prevent the displacement of the stent graft after the stent graft is placed in the blood vessel, and this knotting method can increase the pull-out force of the main stent graft and the branch stent graft. In addition, the patent document US2008/0114443A1 discloses a stent graft with its bare section carrying barbs and a proximal end structure of a transport system for fixing the barbs, in which the bare section and the barb are made by cutting.

Although the knotting method in the patent document EP2301476A1 can improve the pull-out force, the knotting manner is complicated, the time is delayed, and the production efficiency is comparatively low. In the solution of the patent document US2008/0114443A1, there are many barbs of the bare section, two barbs exist on one apex, the barbs are close to each other, and the case that the blood vessel wall is torn and the like may be caused after the placement in the blood vessel. Moreover, in the use of the above stent grafts in the prior art, the positioning and suturing of the stent sections are not convenient, and the bending and adhesion properties of the stent sections are also to be further improved.

European Patent Pub. No. EP2301476A1 discloses modified suture knots which may be utilized to effective anchor one stent-graft to another stent-graft wherein the stent-grafts are normally connected via an interference fit in an overlap configuration. The sutures that are utilized to affix the graft material to the underlying stent structure may be modified to have a profile that allow it to lock and secure it to another stent-graft or a vessel into which it is implanted.

European Patent Pub. No. EP1839624A1 discloses a tubular prosthesis which comprises a tubular member having raised portions, which can be formed from and be part of the tubular member. The raised portions form a chamber or discrete space in a body passageway or lumen between the prosthesis and a portion of the passageway or lumen wall in which it is placed. A substance can be delivered to the chamber to assist the prosthesis placement. The substance can comprise one or more substances that can enhance the seal and/or fixation characteristics between the prosthesis the passageway wall and/or provide therapeutic benefit. In another embodiment, the raised portions can be collars secured to the tubular member and in yet a further embodiment the raised portions can comprise inflatable collars.

U.S. Patent Pub. No. US2003135261A1 discloses an endovascular graft system for repair of aneurysms. The graft system includes a trunk component and first and second leg components. The graft components include graft material supported by a plurality of stents which are spaced apart and affixed to the graft material in a manner that allows articulation of the graft system without excessive wear of the graft material. The stents are formed by intersecting struts which may be tapered to relieve stress. A stabilizing mechanism is provided to stabilize the position of the legs with respect to the trunk when the graft system is deployed.

### SUMMARY OF THE INVENTION

In view of the above technical problems in the prior art, the object of the invention lies in developing a stent graft, which can buffer damages of the apexes of the stent sections on the graft, facilitate positioning and suturing of the stent sections, and improve bending and adhesion properties of the stent sections.

To be specific, the invention provides a stent graft, as defined in claim 1, which comprises: a stent portion including a bare section and a main section; and a graft tube fixed on the main section, wherein the main section includes a plurality of stent sections along its axial direction, characterized in that the stent graft further comprises a plurality of circumferential bosses arranged on an outer circumference of the graft tube along the axial direction, and the circumferential bosses and the stent sections are alternately arranged in the axial direction, and wherein an outer diameter of the circumferential boss is larger than an outer diameter of the graft tube by 1 mm-3 mm, and an axial size of the circumferential boss measured along the axial direction is between 0.5 mm and 1.5 mm, the main section further includes a small wave section, which is arranged close to the bare section, and the small wave section is made of filaments having a diameter of 0.2032-0.254 millimeter, the waveform of the small wave section is designed as a height-changing structure.

Preferably, the plurality of circumferential bosses are arranged on an outer circumference of the graft tube at equal intervals in the axial direction.

Preferably, the bare section includes a plurality of stent rods, which are formed in a wavy structure, at least a part of the bare section is located outside the graft tube, and the bare section further includes a shoulder located at a position of an intersection point of proximal ends of each two adjacent stent rods; a rear release hole connecting to a proximal end of each shoulder, the rear release hole being used for connecting the bare section with a rear release device of a stent transport system; and a barb extending from each shoulder toward a distal end and being located between the corresponding two adjacent stent rods.

Preferably, the rear release hole is an elliptical-like hole, a round hole, a triangular hole, a rectangular hole, a trapezoidal hole or a irregularly-shaped hole.

Preferably, in the case that the shape of the rear release hole is elliptical-like, the rear release hole has a rear release hole length along the axial direction of the stent graft and has a rear release hole width along a circumferential direction of the stent graft, and the thicknesses of two outer walls of the rear release hole in the circumferential direction of the stent graft plus the rear release hole width is an outer profile width, wherein the rear release hole length is between 1.0 mm and 4.0 mm, the rear release hole width is between 0.1 mm and 0.6 mm, and the outer profile width is between 0.8 mm and 1.5 mm.

Preferably, in the case that the stent graft is in an expanded state, the diameter of the proximal end portion of the bare section is larger than the diameter of the distal end portion of the bare section.

Preferably, the thickness of the bare section is between 0.2 mm and 0.8 mm.

Preferably, the thickness of the bare section is between 0.4 mm and 0.6 mm.

Preferably, in the stent portion of the stent graft, only the bare section is a cut stent section, and the other stent sections are all woven stent sections.

Preferably, the stent portion further includes at least two branch sections, and proximal ends of the branch sections are connected with a distal end of the main section.

Preferably, the stent portion further includes a transition section, which is located between the main section and the at least two branch sections and is used for transitioning the main section to the at least two branch sections.

Preferably, the diameter of the proximal end portion of the transition section is the same as the diameter of the distal end portion of the main section, and the transition section tapers gradually from its proximal end portion to its distal end portion to be perform a smooth transition to the at least two branch sections.

According to the invention, the stent graft is provided with the circumferential bosses, which can buffer damages of the apexes of the stent sections on the graft, facilitate positioning and suturing of the stent sections, and improve bending and adhesion properties of the stent sections.

In addition, the transition section adopts a tapered transition shape, and thus can effectively relieve the impact of the blood flow on the graft and the suture lines in the area at the bifurcation; the bare section of the stent graft carries a barb, which can prevent a long-term displacement after the implantation; the proximal end portion of the stent graft is provided with a small wave section, which can improve the adhesion and roundness properties; and with respect to the whole stent graft, the other stent sections than the bare section are all made by weaving, which can reduce the cost and improve the production efficiency.

### BRIEF DESCRIPTION OF DRAWINGS

In order to illustrate the technical solutions of the embodiments of the invention more clearly, the figures to be used in the descriptions of the embodiments will be briefly introduced below. It is obvious that the figures in the descriptions below are only some specific embodiments recorded in the present application, rather than limitations of the scope of protection of the invention. Those skilled in the art certainly can also obtain some other embodiments and figures according to these embodiments and their figures of the invention without making inventive efforts.
Fig. 1 is a schematic diagram of the whole of the stent graft according to one embodiment of the invention;
Fig. 2 is a plan view that shows the structure of the bare section of the stent graft of the invention in an enlarged view;
Fig. 3 is a plan view that shows one structure of the rear release hole;
Fig. 4 is a plan view that shows the other structure of the rear release hole;
Fig. 5 is a schematic diagram of the spatial structure of the bare section after the stent graft of the invention is implanted into the blood vessel of the human body and the bare section is stereotyped;
Fig. 6 is a schematic diagram that shows the structure of the circumferential bosses of the stent graft of the invention;
Fig. 7 is a schematic diagram of the stent graft with the circumferential bosses before bending;
Fig. 8 is a schematic diagram of the stent graft with the circumferential bosses after bending, wherein Fig. 8(a) shows the stent graft in a normal bending form, and Fig. 8(b) shows the stent graft in an ultimate bending form;
Fig. 9 is a schematic diagram that shows the small wave section having an equal-height structure;
Fig. 10 is a schematic diagram that shows the small wave section having a height-changing structure; and
Fig. 11 is an exploded detailed view of the transition section.

### DETAILED DESCRIPTION

In order to make those skilled in the art better understand the technical solutions in the present application, clear and complete descriptions of the technical solutions in the embodiments of the invention will be given below by taking the figures in the embodiments of the invention into consideration. It is obvious that the described embodiments are only parts of the embodiments, rather than all of the embodiments, of the present application. Based on the detailed embodiments of the present application described below, all the other embodiments obtained by those skilled in the art without making inventive efforts shall fall within the scope of the concept of the invention.

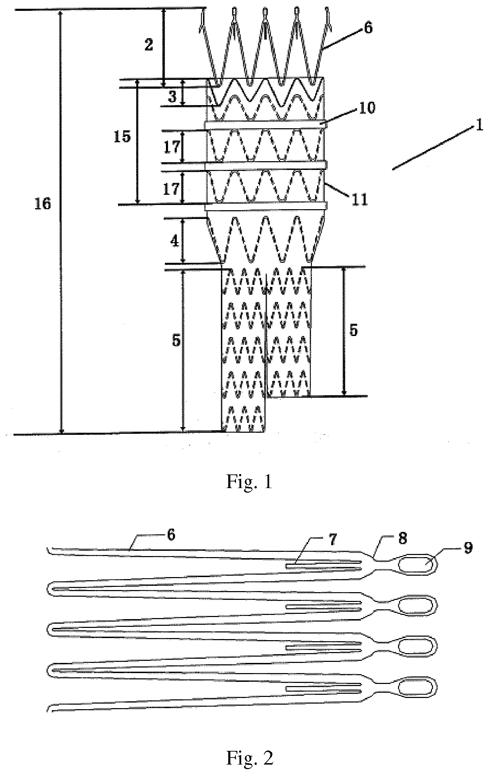

Fig. 1 is a schematic diagram of the whole of the stent graft 1 according to one embodiment of the invention. The stent graft 1 of the invention comprises: a stent portion 16 including a bare section 2 and a main section 15; and a graft tube 11 fixed on the main section 15, wherein the main section 15 includes a plurality of stent sections 17 along its axial direction, and the graft tube 11 is a tubular structure formed of one or more layers of grafts. In the embodiment as shown in Fig. 1, the stent portion 16 includes the bare section 2, the main section 15, a transition section 4 and two branch sections 5 in turn from the proximal end to the distal end of the stent graft 1. The bare section 2 includes a plurality of first stent rods 6, which are formed in a wavy structure, and at least a part of the bare section 2 is located outside the graft tube 11. Each stent section 17 is annularly arranged around the center axis of the stent graft 1. Each stent section 17 consists of a plurality of second stent rods, i.e., the plurality of second stent rods form a ring around the center axis of the stent graft 1 to form one stent section 17. The transition section 4 is located between the main section 15 and the branch sections 5 for transitioning the main section 15 to the branch sections 5. In the embodiment in Fig. 1, only two branch sections 5 of the stent graft are shown, but those skilled in the art should understand that the invention can be also applied to a stent graft having more than two branch sections. In addition, in the invention, the proximal end refers to the end closer to the heart after the stent is implanted into the human body, and the distal end refers to the end farther from the heart after the stent is implanted into the human body. Specifically, in Fig. 1, the proximal end refers to the upper end of the figure, and the distal end refers to the lower end of the figure.

In the stent graft 1 of the invention, in order to prevent a long-term displacement of the stent graft, the bare section 2 at the proximal end of the stent graft is designed as a bare stent structure with a barb 7. Fig. 2 is a plan view that shows the structure of the bare section 2 of the stent graft 1 of the invention in an enlarged view. As shown in Fig. 2, in addition to the first stent rods 6, the bare section 2 further includes a rear release hole 9, a shoulder 8 and a barb 7, the shoulder 8 being located at a position of an intersection point of proximal ends of two adjacent first stent rods 6 and connecting the first stent rods 6 with the rear release hole 9, the rear release hole 9 being used for connecting the bare section 2 with a rear release device of a stent transport system (not shown), and the barb 7 extending from the shoulder 8 toward the distal end and being located between the two adjacent first stent rods 6. In Fig. 2, the proximal end refers to the right end of the figure, and the distal end refers to the left end of the figure.

The proximal end of the bare section 2 is provided with the rear release hole 9, and when the stent graft is released, a rear release device of a stent transport system is used to release the stent graft 1, and the bare section 2 of the stent graft and the rear release device are coupled to each other by a guide wire (not shown) in the rear release device, wherein the guide wire is to pass through the above-described rear release hole 9. The bare section 2 is a cut stent section.

The shoulder 8 of the bare section 2 is used for bearing forces applied from the barb 7 and the rear release hole 9. After the release of the stent graft 1, the barb 7 penetrates into a good blood vessel wall of the human body by means of the tensile force of the bare section 2 to thereby prevent the displacement of the stent graft. The first stent rods 6 form a support structure of the whole stent graft.

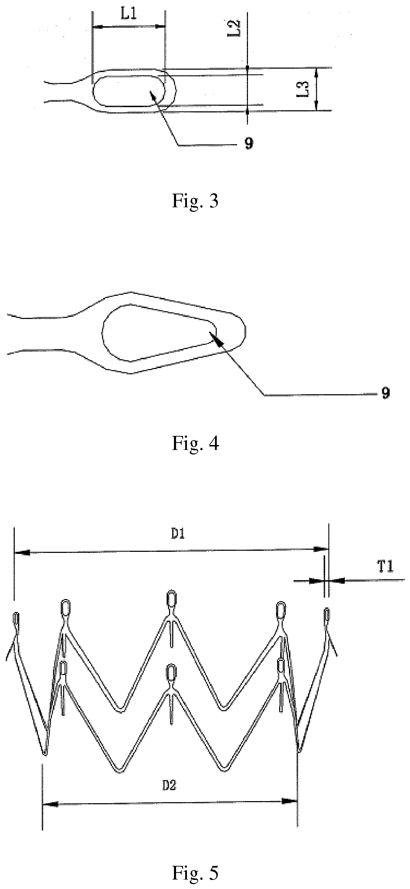

Fig. 3 is a plan view that shows one structure of the rear release hole. As shown in Fig. 3, the rear release hole 9 has an elliptical-like shape. Here, the term "elliptical-like shape" is a common term in the art, and generally refers to a shape similar to the elliptical shape, e.g., the shape shown in Fig. 3, and those skilled in the art can clearly understand the meaning of this term. In Fig. 3, the rear release hole 9 has a rear release hole length L1 along the axial direction of the stent graft and has a rear release hole width L2 along the circumferential direction of the stent graft, and the thicknesses of the two outer walls of the rear release hole 9 in the circumferential direction of the stent graft plus the rear release hole width L2 is an outer profile width L3. The rear release hole length L1 is preferably between 1.0 mm and 4.0 mm. If the rear release hole length L1 is too short, the guide wire may be rendered severely folded after the penetration of the guide wire, which results in that the later release is incomplete or the guide wire cannot be withdrawn; and if the rear release hole length L1 is too long, the length of the whole bare section 2 may be increased, which results in that the lengths of the related components within the matched transport system are increased, thereby increasing the length of the whole transport system. The rear release hole width L2 is preferably between 0.1 mm and 0.6 mm. If the rear release hole width L2 is too narrow, the diameter of the penetrated guide wire may be rendered too thin, so each of the strength of the bare section 2 and the strength of the penetrated guide wire is insufficient, so that the case that the release is incomplete or the guide wire breaks during the release will occur; and if the rear release hole width L2 is too wide, the whole profile of the stent graft may be rendered increased, which results in that the stent graft cannot be mounted in the matched transport system. The outer profile width L3 is preferably between 0.8 mm and 1.5 mm. If the outer profile width L3 is too narrow, the two outer walls of the rear release hole 9 in its transvers direction may be rendered too thin, so the risk of break is likely to occur; and if the outer profile width L3 is too wide, the whole profile of the stent graft may be rendered increased, which results in that the stent graft cannot be mounted in the matched transport system.

Fig. 4 is a plan view that shows the other structure of the rear release hole. A shown in Fig. 4, the rear release hole 9 has a substantially triangular shape. In addition, according to the consideration of the different types and the whole performance of the rear release device, the rear release hole 9 can be also a rectangular hole, a round hole, a trapezoidal hole or a irregularly-shaped hole. The term "irregularly-shaped hole" is a common technical term in the art, and generally refers to a hole different from that of a general type or pattern, e.g., holes having some other irregular shapes than an elliptical-like shape, a round shape, a triangular shape, a rectangular shape and a trapezoidal shape.

Fig. 5 is a schematic diagram of the spatial structure of the bare section after the stent graft of the invention is implanted into the blood vessel of the human body and the bare section is stereotyped. As shown in Fig. 5, after the stent graft 1 is implanted into the blood vessel and the bare section 2 is stereotyped, i.e., in the case that the stent graft 1 is in an expanded state, the bare section 2 forms a ring, and the diameter D1 of the proximal end portion of the bare section 2 is larger than the diameter D2 of the distal end portion of the bare section 2, which facilitates the penetration of the barb 7 into the blood vessel after the stent graft 1 is released from the transport system and opened smoothly to thereby prevent the displacement of the stent graft 1. As shown in Fig. 5, the thickness T1 of the bare section 2 is between 0.2 mm and 0.8 mm, preferably between 0.4 mm and 0.6 mm. If the thickness T1 of the bare section 2 is too thin, the support strength of the bare section 2 will be rendered comparatively weak; and if the thickness T1 of the bare section 2 is too thick, the bare section 2 will be rendered too thick or too hard to thereby result in that the stent graft cannot be opened, and the whole profile of the stent will be rendered increased, which results in a comparatively difficult assembly. It should be noted that in Fig. 5, the thickness of the bare section 2 refers to the thickness size of the bare section 2 in the radial direction of the stent graft 1, the proximal end refers to the upper end of the figure, and the distal end refers to the lower end of the figure.

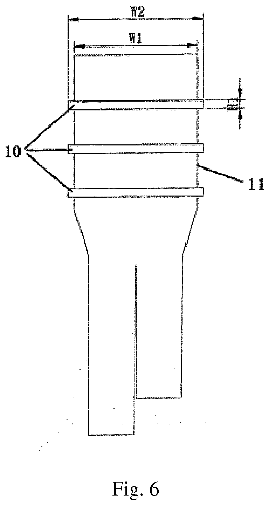

Fig. 6 is a schematic diagram that shows the structure of the circumferential bosses of the stent graft of the invention. As shown in Fig. 1 and Fig. 6, in the stent graft 1, a plurality of circumferential bosses 10 integrated with the graft tube 11 are formed (three circumferential bosses are shown in the embodiments in Fig. 1 and Fig. 6) outside the graft tube 11, namely the material of the circumferential bosses 10 is the same as the material of the graft tube 11, both of which are flexible materials. Each of the plurality of circumferential bosses 10 extends around the outer circumferential surface of the graft tube 11 in the circumferential direction and protrudes towards the outer side of the graft tube 11. The circumferential bosses 10 and the stent sections 17 of the main section 15 are alternately arranged in the axial direction of the main section 15. Preferably, the plurality of circumferential bosses 10 are arranged on the outer circumference of the graft tube 11 at equal intervals in the axial direction of the main section 15. But, those skilled in the art can understand that in some other embodiments of the invention, the spacing distances between the plurality of circumferential bosses 10 may be also not equal.

The circumferential bosses 10 outside the graft of the invention have the following characteristics:

### ① Relieving the fatigue wear of the apex of the stent section

When the stent graft is axially compressed, if there are no circumferential bosses 10, the compressions between the stent sections 17 will be irregular, and since the support strength of the stent section is large, the impact of the apex of the stent section on the graft(s) of the graft tube 11 is comparatively strong, and a long-term wear may result in that the apex of the stent section bursts the graft, thus the stent graft may be rendered leaky; and after the addition of the circumferential bosses 10, the whole stent graft will be compressed along the trace of the circumferential bosses 10 after the stent graft is applied by an axial compression force, so the wear of the stent graft by the apex of the stent section may be relieved.

### ② Adding adhesion properties

After the stent graft is implanted into the human body, the case that there is a plaque on the blood vessel wall at the implantation site may be encountered, and after the part having a metal stent meets the plaque, adverse consequences such as a bad adhesion and an internal leakage are likely resulted in. In contrast, after the stent graft 1 with the circumferential bosses 10 of the invention is implanted into the blood vessel tumor cavity, along with the impact of the blood flow, the contact of the graft material of a circumferential boss structure with the blood vessel wall will be increased, so the adhesion properties of the whole stent graft may be increased and the internal leakage may be reduced.

### ③ Facilitating bending and increasing smoothness of the blood flow

The stent graft 1 of the invention is provided with a plurality of circumferential bosses 10, so the whole of the stent graft 1 is bent along with the shape of the circumferential bosses 10 when the stent graft 1 is bent. Thus, the bending properties of the whole of the stent graft may be increased after the suturing of the stent sections.

Generally, the outer diameter W2 of the circumferential boss 10 is larger than the outer diameter W1 of the graft tube 11 of the stent graft 1 by 1 mm-3 mm (see Fig. 6), and the width HI of the circumferential boss 10 is between 0.5 mm and 1.5 mm. If the width HI of the circumferential boss 10 is too narrow, the circumferential boss 10 may be rendered unable to effectively reduce the wear between the stent section and the graft and achieve the effect of bending flexibility; and if the width HI of the circumferential boss 10 is too wide, the interval between the stent sections may be rendered too large, which, on the contrary, is more likely to cause folding. Here, the outer diameter of the circumferential boss 10 refers to the diameter of the outermost part of the circumferential boss 10 extending around the center axis of the stent graft 1, and the width of the circumferential boss 10 refers to the axial size of the circumferential boss 10 measured along the center axis of the stent graft 1.

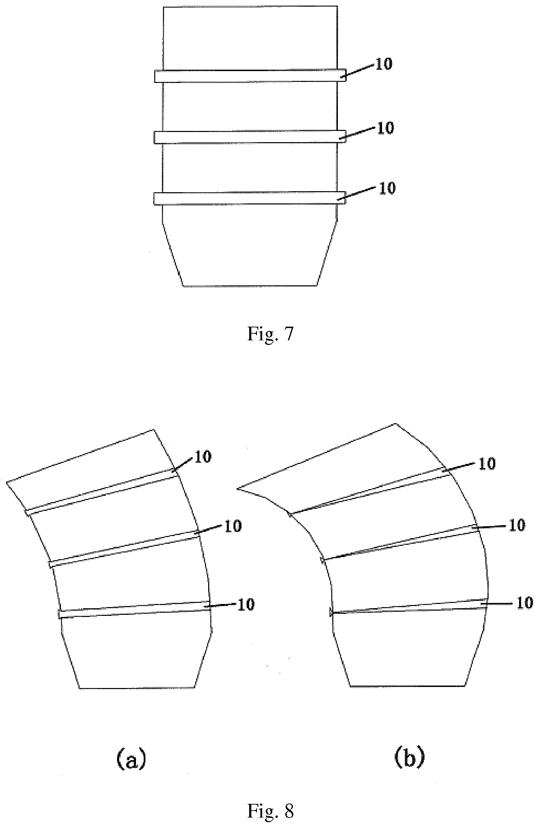

The forms of the stent graft 1 with the circumferential bosses 10 before and after bending are shown in Fig. 7 and Fig. 8. Fig. 7 is a schematic diagram of the stent graft with the circumferential bosses 10 before bending, wherein the graft tube of the stent graft is straight. Fig. 8 is a schematic diagram of the stent graft with the circumferential bosses 10 after bending, wherein the graft tube of the stent graft 1 is bent, wherein Fig. 8(a) shows the stent graft 1 in a normal bending form, and Fig. 8(b) shows the stent graft 1 in an ultimate bending form.

As shown in Fig. 7 and Fig. 8, when the graft tube 11 with the circumferential bosses 10 is bent, the graft tube 11 will be bent along the trace of the circumferential bosses, and along with the increase of the bending degree, the lesser bending side (the left side in Fig. 8(b), i.e., the inner side of the bent part) of the bending of the circumferential boss 10 is compressed, and the upper edge and lower edge in the circumferential boss 10 contact with each other along with the deepening of the bending degree, as shown in Fig. 8(b), in which case since the circumferential bosses 10 are located at the outer side of the whole stent graft, they will not influence the blood flow; and the greater bending side (the right side in Fig. 8(b), i.e., the outer side of the bent part) of the bending of the circumferential boss 10 will be completely expanded to facilitate the increase of its bending amount. If there are no circumferential bosses 10, in the case that the greater bending side of the graft is completely expanded, the graft at the lesser bending side will be inevitably wrinkled, and the wrinkle inside the stent graft will influence the speed of the blood flow. In contrast, in the invention, with respect to the lesser bending side with the circumferential bosses 10, along with the increase of the bending degree, the upper edge and lower edge in the circumferential boss 10 at the lesser bending side will contact with each other, and the other grafts are supported by the stent sections, so no excess wrinkles will be produced in the internal grafts to influence the blood flow.

### ④ Accurately positioning the stent sections

The addition of the circumferential bosses 10 may facilitate the positioning of the stent sections and facilitate precise suturing, so that the practicability of the suturing process is greatly increased.

All the stent sections for treating the abdominal aortic aneurysm have comparatively large diameters, a large-scale cutting machine is comparatively expensive, and some after-treatment operations, e.g., polishing, stereotyping, sand blasting, or other subsequent processing operations, are to be performed after the cutting of the stent sections, so the price for cutting the stent sections is high. The woven stent sections can stereotype the desired outlines of the stent sections only by various weaving and stereotyping molds, i.e., the adoption of the woven stent sections can both reduce the cost and ensure the same performance. In the stent graft 1 of the invention, it is allowed to form only the bare section 2 into a cut stent section and form all the other stent sections, including the aforementioned wave section 3 and stent section 17, as well as the sections in the branch sections 5 and the central structure of the transition section to be mentioned below, into woven stent sections, thereby reducing the cost.

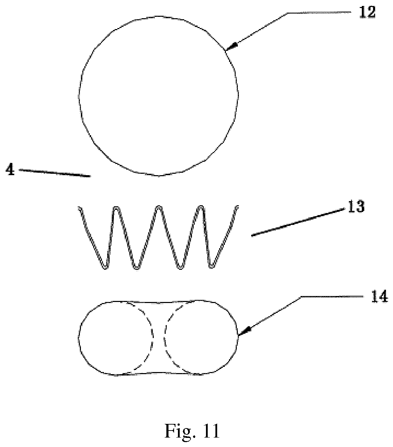

As shown in Fig. 1, the stent portion 16 further includes at least two branch sections 5, and the proximal ends of the branch sections 5 are connected with the distal end of the main section 15. The stent portion 16 further includes a transition section 4, which is located between the main section 15 and the branch sections 5 and is used for transitioning the main section 15 to the branch sections 5. The design of the transition section 4 in the stent portion 16 of the stent graft is as shown in Fig. 11. Fig. 11 is an exploded detailed view of the transition section 4. Since the diameter of the main section 15 of the stent graft 1 is comparatively large, and the diameter of the branch section 5 is comparatively small, if the connection is directly made from the main section of the stent graft 1 to the branch sections, the impact of the blood flow on the graft at the connection section, especially the graft at the bifurcation and the neighboring suture lines, will be very large, which will result in that the graft in the area at the bifurcation is severely impacted by the blood flow to be damaged, and which will result in a severe wear at the suture of the main section and the first section of the stent sections of the branch sections, so the stent sections may drop or leakage of blood may occur at the bifurcation, and thereby cause a severe result.

With respect to the above problem, the invention designs a transition section 4, which can smoothly and stably transition the part having a large diameter of the stent graft at the proximal end to the parts having small diameters of the two branch sections at the distal end. As shown in Fig. 1 and Fig. 11, the diameter of the proximal end portion 12 of the transition section 4 is the same as the diameter of the distal end portion of the main section 15, and the transition section 4 tapers gradually from its proximal end portion 12 to its distal end portion 14 to perform a smooth transition to the two branch sections 5, where the reference sign 13 denotes a central structure of the transition section. It should be noted that in Fig. 11, the proximal end refers to the upper end of the figure, and the distal end refers to the lower end of the figure. By the above design of the invention, the influence of the impact of the blood flow on the stent graft can be reduced after the whole stent graft is implanted.

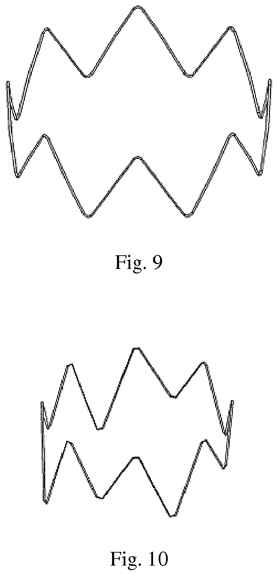

In the invention, a small wave section 3 having a woven structure increase the adhesion and roundness properties of the proximal end of the stent graft. As shown in Fig. 1, the main section 15 further include a small wave section 3, which is arranged close to the bare section 2. In Fig. 1, the small wave section 3 is located at the edge portion the proximal end of the graft tube 11 formed of the graft material. The small wave section 3 is used for improving the adhesion properties of the proximal end of the stent graft and preventing the generation of the internal leakage. The waveform structure of the small wave section 3 is dimensionally smaller than the waveform structures of the other stent sections in the stent portion 16. The waveform of the small wave section 3 is designed as an equal-height structure or a height-changing structure. The filament diameter of the small wave section is comparatively thin. The structure of the small wave section is as shown in Fig. 9 and Fig. 10. Fig. 9 is a schematic diagram that shows the small wave section 3 having an equal-height structure. The small wave section 3 has an equal-height structure, i.e., the structure in which all the upper apexes are on the same plane, all the lower apexes are on the other same plane, and the respective stent rods have the same length is called an equal-height structure. Fig. 10 is a schematic diagram that shows the small wave section 3 having a height-changing structure. In the height-changing structure of the small wave section 3 shown in Fig. 10, all the upper apexes are on the same plane, but not all the lower apexes are on the other same plane, and this height-changing structure having apexes being not on the same plane facilitates reduction of the profile of the whole stent graft.

Since the function of the small wave section 3 is to increase the adhesion and roundness properties, the filament diameter of the small wave section 3 is selected to be comparatively thin, and a filament diameter of 0.2032-0.254 millimeter (0.008-0.010 inch), which is greatly smaller than the filament diameter of the stent sections of the main section, is generally selected.

The stent graft of the invention is adapted to treat the abdominal aortic aneurysm. During the operation, the stent graft is released after being guided to the lesion site, the stent made of metal material supports the graft material on the normal blood vessel walls at the both ends of the diseased blood vessel, so that the stent graft isolates the aneurysm from the blood flow within the blood vessel cavity to thereby eliminate the risk of break of the blood vessel and achieve the object of maintaining the blood flow of the aorta smooth; and the outer wall of the blood vessel is retracted due to the negative pressure, the normal blood vessel shape can be slowly restored, and the vascular intima cells permeate the micropores in the graft material and finally completely cover the stent graft to achieve endothelialization of the stent graft. Moreover, the invention can be applied to the cases of the abdominal aortic aneurysm involving a short tumor neck and a bent tumor neck, i.e., the stent graft of the invention is more suitable for a comparatively short tumor neck length and a tumor neck that is bent more, has better adhesion and roundness properties, has a moderate support strength, can prevent a long-term displacement of the stent graft after the implantation of the stent graft and the like.

As compared with the stent grafts in the prior art, the stent graft of the invention has the following advantageous technical effects:
1. The stent graft is provided with the circumferential bosses, which can buffer damages of the apexes of the stent sections on the graft, facilitate positioning and suturing of the stent sections, and improve bending and adhesion properties;
2. The transition section adopts a tapered transition shape, and thus can effectively relieve the impact of the blood flow on the graft and the suture lines in the area at the bifurcation;
3. The bare section of the stent graft carries a barb, which can prevent a long-term displacement after the implantation;
4. The proximal end portion of the stent graft is provided with a small wave section, which can improve the adhesion and roundness properties; and
5. With respect to the whole stent graft, the other stent sections than the bare section are all made by weaving, which can reduce the cost and improve the production efficiency.

The stent graft of the invention can meet the various performance indexes to evaluate this kind of stent grafts as mentioned above in the Description, i.e., the stent graft has a very good tightness and thus can isolate the tumor cavity from the blood; the bare section carries a barb, and thus can prevent the displacement of the stent after the implantation of the stent; the adoption of a proper release resistance can smoothly release the stent and avoid the displacement of the stent; the rear release function of the stent can accurately position the stent graft; the stent graft has a comparatively low edge height and thus has a good flexibility; and the stent graft can be adapted to a smaller tumor neck length. To sum up, the invention can be adapted to various types of abdominal aortic aneurysms.

The above contents are only some detailed embodiments of the present application. It should be noted that those skilled in the art can also make various combinations or make some improvements and variations with respect to the above embodiments in the case of not breaking away from the inventive principle and inventive concept of the present application, and these combinations, improvements and variations should be also deemed as ones falling within the scope of protection and the inventive concept of the present application as long as covered by the claims language.

## Claims

1. A stent graft (1), comprising: a stent portion (16) including a bare section (2) and a main section (15); and a graft tube (11) fixed on the main section (15), wherein the main section (15) includes a plurality of stent sections (17) along its axial direction,
the stent graft (1) further comprises a plurality of circumferential bosses (10) arranged on an outer circumference of the graft tube (11) along the axial direction, and the circumferential bosses (10) and the stent sections (17) are alternately arranged in the axial direction, **characterized in that**
an outer diameter (W2) of the circumferential boss (10) is larger than an outer diameter (W1) of the graft tube (11) by 1 mm-3 mm, and an axial size of the circumferential boss (10) measured along the axial direction is between 0.5 mm and 1.5 mm,
the main section (15) further includes a small wave section (3), which is arranged close to the bare section (2), and the small wave section (3) is made of filaments having a diameter of 0.2032-0.254 millimeter,
the waveform of the small wave section (3) is designed as a height-changing structure.

2. The stent graft (1) according to claim 1, **characterized in that** the plurality of circumferential bosses (10) are arranged on an outer circumference of the graft tube (11) at equal intervals in the axial direction.

3. The stent graft (1) according to claim 1 or 2, **characterized in that**:
the bare section (2) includes a plurality of stent rods (6), which are formed in a wavy structure, at least a part of the bare section (2) is located outside the graft tube (11), and the bare section (2) further includes:
a shoulder (8) located at a position of an intersection point of proximal ends of each two adjacent stent rods (6);
a rear release hole (9) connecting to a proximal end of each shoulder (8) , the rear release hole (9) being used for connecting the bare section (2) with a rear release device of a stent transport system; and
a barb (7) extending from each shoulder (8) toward a distal end and being located between the corresponding two adjacent stent rods (6).

4. The stent graft (1) according to claim 3, **characterized in that** the rear release hole (9) is an elliptical-like hole, a round hole, a triangular hole, a rectangular hole, a trapezoidal hole or a irregularly-shaped hole; in the case that the shape of the rear release hole (9) is elliptical-like, the rear release hole (9) has a rear release hole length (L1) along the axial direction of the stent graft and has a rear release hole width (L2) along a circumferential direction of the stent graft, and the thicknesses of two outer walls of the rear release hole (9) in the circumferential direction of the stent graft plus the rear release hole width (L2) is an outer profile width (L3), wherein the rear release hole length (L1) is between 1.0 mm and 4.0 mm, the rear release hole width (L2) is between 0.1 mm and 0.6 mm, and the outer profile width (L3) is between 0.8 mm and 1.5 mm.

5. The stent graft (1) according to claim 1 or 2, **characterized in that** in the case that the stent graft (1) is in an expanded state, the diameter (D1) of the proximal end portion of the bare section (2) is larger than the diameter (D2) of the distal end portion of the bare section (2).

6. The stent graft (1) according to claim 1 or 2, **characterized in that** the thickness (T1) of the bare section (2) is between 0.2 mm and 0.8 mm.

7. The stent graft (1) according to claim 6, **characterized in that** the thickness (T1) of the bare section (2) is between 0.4 mm and 0.6 mm.

8. The stent graft (1) according to claim 1 or 2, **characterized in that** in the stent portion (16) of the stent graft (1), only the bare section (2) is a cut stent section, and the other stent sections are all woven stent sections.

9. The stent graft (1) according to claim 1 or 2, **characterized in that** the stent portion (16) further includes at least two branch sections (5), and proximal ends of the branch sections (5) are connected with a distal end of the main section (15).

10. The stent graft (1) according to claim 9, **characterized in that** the stent portion (16) further includes a transition section (4), which is located between the main section (15) and the at least two branch sections (5) and is used for transitioning the main section (15) to the at least two branch sections (5).

11. The stent graft (1) according to claim 10, **characterized in that** the diameter of the proximal end portion (12) of the transition section (4) is the same as the diameter of the distal end portion of the main section (15), and the transition section (4) tapers gradually from its proximal end portion (12) to its distal end portion (14) to perform a smooth transition to the at least two branch sections (5).

## Patentansprüche

1. Stenttransplantat (1), umfassend: einen Stentabschnitt (16), der einen blanken Abschnitt (2) und einen Hauptabschnitt (15) beinhaltet; und ein Transplantatrohr (11), das an dem Hauptabschnitt (15) befestigt ist, wobei der Hauptabschnitt (15) eine Vielzahl von Stentabschnitten (17) entlang seiner axialen Richtung beinhaltet,
wobei das Stenttransplantat (1) ferner eine Vielzahl von Umfangsvorsprüngen (10) umfasst, die an einem Außenumfang des Transplantatrohrs (11) entlang der axialen Richtung angeordnet sind, und die Umfangsvorsprünge (10) und die Stentabschnitte (17) abwechselnd in der axialen Richtung angeordnet sind, **dadurch gekennzeichnet, dass**
ein Außendurchmesser (W2) des Umfangsvorsprungs (10) um 1 mm - 3 mm größer ist als ein Außendurchmesser (W1) des Transplantatrohrs (11), und eine axiale Größe des Umfangsvorsprungs (10), gemessen entlang der axialen Richtung, zwischen 0.5 mm und 1.5 mm ist,
der Hauptabschnitt (15) ferner einen kleinen Wellenabschnitt (3) beinhaltet, der nahe dem blanken Abschnitt (2) angeordnet ist, und der kleine Wellenabschnitt (3) aus Filamenten mit einem Durchmesser von 0.2032-0.254 Millimeter gefertigt ist,
die Wellenform des kleinen Wellenabschnitts (3) ist als höhenveränderliche Struktur konstruiert ist.

2. Stenttransplantat (1) nach Anspruch 1, **dadurch gekennzeichnet, dass** die Vielzahl von Umfangsvorsprüngen (10) in gleichen Abständen in axialer Richtung an einem Außenumfang des Transplantatrohrs (11) angeordnet sind.

3. Stenttransplantat (1) nach Anspruch 1 oder 2, **dadurch gekennzeichnet, dass**:
der blanke Abschnitt (2) eine Vielzahl von Stentstäben (6) beinhaltet, die in einer wellenförmigen Struktur gebildet sind, mindestens ein Teil des blanken Abschnitts (2) sich außerhalb des Transplantatrohrs (11) befindet und der blanke Abschnitt (2) ferner Folgendes beinhaltet:
eine Schulter (8), die sich an einer Position eines Überschneidungspunkts von proximalen Enden von zwei benachbarten Stentstäben (6) befinden;
ein hinteres Freigabeloch (9), das mit einem proximalen Ende von jeder Schulter (8) verbunden ist, wobei das hintere Freigabeloch (9) zum Verbinden des blanken Abschnitts (2) mit einer hinteren Freigabevorrichtung eines Stenttransportsystems verwendet wird; und
einen Widerhaken (7), der sich von jeder Schulter (8) in Richtung eines distalen Endes erstreckt und sich zwischen den entsprechenden zwei benachbarten Stentstäben (6) befindet.

4. Stenttransplantat (1) nach Anspruch 3, **dadurch gekennzeichnet, dass** das hintere Freigabeloch (9) ein ellipsenförmiges Loch, ein rundes Loch, ein dreieckiges Loch, ein rechteckiges Loch, ein trapezförmiges Loch oder ein unregelmäßig geformtes Loch ist; in dem Fall, dass die Form des hinteren Freigabelochs (9) elliptisch ist, das hintere Freigabeloch (9) eine hintere Freigabelochlänge (L1) entlang der axialen Richtung des Stenttransplantats und eine hintere Freigabelochbreite (L2) entlang einer Umfangsrichtung des Stenttransplantats aufweist, und die Stärken von zwei Außenwänden des hinteren Freigabelochs (9) in der Umfangsrichtung des Stenttransplantats plus der hinteren Freigabelochbreite (L2) eine äußere Profilbreite (L3) ist, wobei die hintere Freigabelochlänge (L1) zwischen 1.0 mm und 4.0 mm ist, die hintere Freigabelochbreite (L2) zwischen 0.1 mm und 0.6 mm ist und die äußere Profilbreite (L3) zwischen 0.8 mm und 1.5 mm ist.

5. Stenttransplantat (1) nach Anspruch 1 oder 2, **dadurch gekennzeichnet, dass** in dem Fall, dass das Stenttransplantat (1) in einem expandierten Zustand ist, der Durchmesser (D1) des proximalen Endabschnitts des blanken Abschnitts (2) größer ist als der Durchmesser (D2) des distalen Endabschnitts des blanken Abschnitts (2).

6. Stenttransplantat (1) nach Anspruch 1 oder 2, **dadurch gekennzeichnet, dass** die Stärke (T1) des blanken Abschnitts (2) zwischen 0,2 mm und 0,8 mm ist.

7. Stenttransplantat (1) nach Anspruch 6, **dadurch gekennzeichnet, dass** die Stärke (T1) des blanken Abschnitts (2) zwischen 0,4 mm und 0,6 mm ist.

8. Stenttransplantat (1) nach Anspruch 1 oder 2, **dadurch gekennzeichnet, dass** in dem Stentabschnitt (16) des Stenttransplantats (1) nur der blanke Abschnitt (2) ein geschnittener Stentabschnitt ist und die anderen Stentabschnitte alle gewebte Stentabschnitte sind.

9. Stenttransplantat (1) nach Anspruch 1 oder 2, **dadurch gekennzeichnet, dass** der Stentabschnitt (16) ferner mindestens zwei Verzweigungsabschnitte (5) beinhaltet, wobei die proximalen Enden der Verzweigungsabschnitte (5) mit einem distalen Ende des Hauptabschnitts (15) verbunden sind.

10. Stenttransplantat (1) nach Anspruch 9, **dadurch gekennzeichnet, dass** der Stentabschnitt (16) ferner einen Übergangsabschnitt (4) beinhaltet, der sich zwischen dem Hauptabschnitt (15) und den mindestens zwei Verzweigungsabschnitten (5) befindet und zum Übergang des Hauptabschnitts (15) auf die mindestens zwei Verzweigungsabschnitte (5) verwendet wird.

11. Stenttransplantat (1) nach Anspruch 10, **dadurch gekennzeichnet, dass** der Durchmesser des proximalen Endabschnitts (12) des Übergangsabschnitts (4) gleich ist wie der Durchmesser des distalen Endabschnitts des Hauptabschnitts (15), und dass sich der Übergangsabschnitt (4) von seinem proximalen Endabschnitt (12) zu seinem distalen Endabschnitt (14) allmählich verjüngt, um einen sanften Übergang zu den mindestens zwei Verzweigungsabschnitten (5) auszuführen.

## Revendications

1. Endoprothèse (1), comprenant: une partie d'endoprothèse (16) comprenant une section nue (2) et une section principale (15) ; et un tube de greffe (11) fixé à la section principale (15), dans laquelle la section principale (15) comprend une pluralité de sections d'endoprothèse (17) le long de sa direction axiale,
l'endoprothèse (1) comprend en outre une pluralité de bossages circonférentiels (10) disposés sur une circonférence extérieure du tube de greffe (11) le long de la direction axiale, et les bossages circonférentiels (10) et les sections d'endoprothèse (17) sont disposés alternativement dans la direction axiale, **caractérisée en ce que**
un diamètre extérieur (W2) du bossage circonférentiel (10) est supérieur à un diamètre extérieur (W1) du tube de greffe (11) de 1 mm à 3 mm, et une taille axiale du bossage circonférentiel (10) mesurée le long de la direction axiale est comprise entre 0,5 mm et 1,5 mm,
la section principale (15) comprend en outre une section de petites ondes (3), qui est disposée à proximité de la section nue (2), et la section de petites ondes (3) est constituée de filaments ayant un diamètre de 0,2032 à 0,254 millimètre,
la forme d'onde de la section de petites ondes (3) est conçue comme une structure à changement de hauteur.

2. Endoprothèse (1) selon la revendication 1, **caractérisée en ce que** la pluralité de bossages circonférentiels (10) sont disposés sur une circonférence extérieure du tube de greffe (11) à intervalles réguliers dans la direction axiale.

3. Endoprothèse (1) selon la revendication 1 ou 2, **caractérisée en ce que** :
la section nue (2) comprend une pluralité de tiges d'endoprothèse (6), qui sont formées dans une structure ondulée, au moins une partie de la section nue (2) est située à l'extérieur du tube de greffe (11), et la section nue (2) comprend en outre :
un épaulement (8) situé au niveau d'un point d'intersection des extrémités proximales de chaque pair de tiges d'endoprothèse adjacentes (6) ;
un trou de libération arrière (9) relié à une extrémité proximale de chaque épaulement (8), le trou de libération arrière (9) étant utilisé pour relier la section nue (2) à un dispositif de libération arrière d'un système de transport d'endoprothèse ; et
un ardillon (7) s'étendant depuis chaque épaulement (8) vers une extrémité distale et étant situé entre les deux tiges d'endoprothèse adjacentes (6) correspondantes.

4. Endoprothèse (1) selon la revendication 3, **caractérisée en ce que** le trou de libération arrière (9) est un trou de type elliptique, un trou rond, un trou triangulaire, un trou rectangulaire, un trou trapézoïdal ou un trou de forme irrégulière ; dans le cas où la forme du trou de libération arrière (9) est de type elliptique, le trou de libération arrière (9) présente une longueur de trou de libération arrière (L1) le long de la direction axiale de l'endoprothèse et présente une largeur de trou de libération arrière (L2) le long d'une direction circonférentielle de l'endoprothèse, et les épaisseurs de deux parois extérieures du trou de libération arrière (9) dans la direction circonférentielle de l'endoprothèse plus la largeur de trou de libération arrière (L2) est une largeur de profil extérieur (L3), dans laquelle la longueur de trou de libération arrière (L1) est comprise entre 1,0 mm et 4,0 mm, la largeur de trou de libération arrière (L2) est comprise entre 0,1 mm et 0,6 mm, et la largeur de profil extérieur (L3) est comprise entre 0,8 mm et 1,5 mm.

5. Endoprothèse (1) selon la revendication 1 ou 2, **caractérisée en ce que**, dans le cas où l'endoprothèse (1) est dans un état déployé, le diamètre (D1) de la partie d'extrémité proximale de la section nue (2) est supérieur au diamètre (D2) de la partie d'extrémité distale de la section nue (2).

6. Endoprothèse (1) selon la revendication 1 ou 2, **caractérisée en ce que** l'épaisseur (T1) de la section nue (2) est comprise entre 0,2 mm et 0,8 mm.

7. Endoprothèse (1) selon la revendication 6, **caractérisée en ce que** l'épaisseur (T1) de la section nue (2) est comprise entre 0,4 mm et 0,6 mm.

8. Endoprothèse (1) selon la revendication 1 ou 2, **caractérisée en ce que** dans la partie d'endoprothèse (16) de l'endoprothèse (1), seule la section nue (2) est une section d'endoprothèse découpée, et les autres sections d'endoprothèse sont toutes des sections d'endoprothèse tissées.

9. Endoprothèse (1) selon la revendication 1 ou 2, **caractérisée en ce que** la partie d'endoprothèse (16) comprend en outre au moins deux sections de ramification (5), et les extrémités proximales des sections de ramification (5) sont reliées à une extrémité distale de la section principale (15).

10. Endoprothèse (1) selon la revendication 9, **caractérisée en ce que** la partie d'endoprothèse (16) comprend en outre une section de transition (4), qui est située entre la section principale (15) et les au moins deux sections de ramification (5) et qui est utilisée pour faire la transition entre la section principale (15) et les au moins deux sections de ramification (5).

11. Endoprothèse (1) selon la revendication 10, **caractérisée en ce que** le diamètre de la partie d'extrémité proximale (12) de la section de transition (4) est identique au diamètre de la partie d'extrémité distale de la section principale (15), et la section de transition (4) se rétrécit progressivement depuis sa partie d'extrémité proximale (12) jusqu'à sa partie d'extrémité distale (14) pour réaliser une transition douce vers les au moins deux sections de ramification (5).
